# EUROPEAN PATENT APPLICATION

(11) **EP 4 790 060 A2**
(43) Date of publication of application: **12.08.2026**
(21) Application number: 25225745.6
(22) Date of filing: 19.12.2025
(51) Int. Cl.: C07H 1/00, C07H 19/207, C12Q 1/00, G01N 27/327

(54) **NAD(P) MODIFICATION METHOD, IMMOBILIZATION METHOD, AND CONTINUOUS ANALYSIS SENSOR**

(30) Priority: 16.01.2025 CN 202510069298
(71) Applicant: Changsha Sinocare Inc., Changsha (CN)
(72) Inventor: GAO, Fei, Changsha (CN); LUO, Jinqiu, Changsha (CN); MIN, Xiaofang, Changsha (CN)
(74) Representative: Monteiro Alves, Inês

(57) **Abstract**

A NAD(P) modification method, comprising: S1: using Br-(CH₂)ₓ-NH₂·HBr to react with NAD(P), thereby obtaining N¹-(x-(CH₂)ₓNH₂)-NAD(P)⁺; and S2: performing rearrangement reaction on N¹-(x-(CH₂)ₓNH₂)-NAD(P)⁺, thereby obtaining N⁶-(x-(CH₂)ₓNH₂)-NAD(P)⁺, wherein x=2-6. The present invention also provides a NAD(P) immobilization method and a continuous analysis sensor. The method is environmentally friendly and harmless. By means of adding an amino group to NAD(P), effective immobilization of NAD(P) is achieved, so that the stability of the sensor is significantly improved and the functional life of the sensor is greatly prolonged.

## Description

### TECHNICAL FIELD

This invention generally relates to the technical field of biosensor technology, and more particularly, to a NAD(P) modification method, an immobilization method, and a continuous analysis sensor.

### BACKGROUND OF THE INVENTION

Diabetes is the most common endocrine disorder disease in clinical practice. The etiology and pathogenesis of diabetes are complex. This disease is primarily characterized by the ineffective utilization and storage of glucose in the blood, leading to prolonged high blood sugar levels. High blood sugar levels over a prolonged period of time may cause functional damage to tissues and organs, including the heart, kidneys and eyes, and may lead to diabetic ketosis (DK) or diabetic ketoacidosis (DKA) due to stress or improper treatment. Monitoring the concentration of β-hydroxybutyrate in interstitial fluid can effectively reflect variations of blood ketone levels, thereby providing an early warning of the risk of diabetic ketosis or diabetic ketoacidosis and enabling a timely and accurate intervention.

In the prior art, sensor systems are employed to monitor diabetes-related physiological indicators, such as blood glucose and blood ketones (β-hydroxybutyrate). Taking the blood ketone indicator as an example, the sensor system for detecting blood ketones comprises a working electrode, a β-hydroxybutyrate enzyme-sensing layer arranged on the working electrode, and a β-hydroxybutyrate polymer restriction membrane arranged on the enzyme-sensing layer. The enzyme-sensing layer mainly comprises β-hydroxybutyrate dehydrogenase that responds to β-hydroxybutyrate, a coenzyme nicotinamide adenine dinucleotide (NAD) or nicotinamide adenine dinucleotide phosphate (NADP) that is operatively in contact with the β-hydroxybutyrate dehydrogenase, diaphorase that enables the regeneration of NAD(P), and an electron mediator.

NAD and NADP primarily serve as coenzymes for dehydrogenases, acting as hydrogen carriers (single hydrogen carriers) in enzymatic reactions. They are small organic molecules, non-protein compounds, and loosely bind to enzymes. They do not directly enhance the catalytic capability of enzymes but participate in the catalytic reaction together with the enzymes. However, during the continuous monitoring of β-hydroxybutyrate, NAD(P) gradually permeates through the polymer restriction membrane over time, which has a certain impact on the catalytic reaction of the sensor and thus affect the performance of the sensor.

Reducing the permeation of NAD(P) to minimize its impact on sensor performance is a pressing technical challenge that needs to be addressed those skilled in the art.

### SUMMARY OF THE INVENTION

The purpose of the present invention is to provide a NAD(P) modification method, a modified NAD(P) immobilization method, and a continuous analysis sensor.

To achieve the above purpose, the present invention adopts the following technical solution: a NAD(P) modification method, comprising:
S1: using Br-(CH₂)ₓ-NH₂·HBr to react with NAD(P), thereby obtaining N¹-(x-(CH₂)ₓNH₂)-NAD(P)⁺;
S2: performing rearrangement reaction of N¹-(x-(CH₂)ₓNH₂)-NAD(P)⁺, thereby obtaining N⁶-(x-(CH₂)ₓNH₂)-NAD(P)⁺, wherein x=2-6.

In another preferred embodiment, in S1, respectively dissolving Br-(CH₂)ₓ-NH₂·HBr and NAD(P) in a first solvent; subsequently, dropwise adding the Br-(CH₂)ₓ-NH₂·HBr solution into the NAD(P) solution; reacting at a temperature of 20-30°C for 18-24 hours under the protection of inert gas, and separating, thereby obtaining N¹-(x-(CH₂)ₓNH₂)-NAD(P)⁺.

In another preferred embodiment, the first solvent is water and/or methanol, and the inert gas is nitrogen or helium.

In another preferred embodiment, after the reaction is completed, performing vacuum rotary evaporation to remove solvent, washing with ethanol for 2-3 times, centrifuging for precipitation, and performing vacuum rotary for drying; after performing ion exchange chromatography, obtaining N¹-(x-(CH₂)ₓNH₂)-NAD(P)⁺.

In another preferred embodiment, in S2, dissolving N¹-(x-(CH₂)ₓNH₂)-NAD(P)⁺ in water, adding into 1-5mM LiOH solution, adjusting the pH to 6.4-6.6, reacting at a temperature of 45-55°C for 4-6 hours, and separating, thereby obtaining N⁶-(x-(CH₂)ₓNH₂)-NAD(P)⁺.

In another preferred embodiment, after the reaction is completed, performing ion exchange chromatography, and then performing vacuum rotary evaporation for drying, thereby obtaining N⁶-(x-(CH₂)ₓNH₂)-NAD(P)⁺.

In another preferred embodiment, Br-(CH₂)ₓ-NH₂·HBr is specifically any one of bromoethylamine hydrobromide, bromopropylamine hydrobromide, bromobutanamine hydrobromide, bromopentylamine hydrobromide, and bromhexylamine hydrobromide.

A modified NAD(P) immobilization method, comprising: coupling and immobilizing N⁶-(x-(CH₂)ₓNH₂)-NAD(P)⁺ to a carrier material having amino or carboxyl functional groups, wherein x=2-6.

In another preferred embodiment, the carrier material is any one of polylysine, polyethyleneimine, polylactic acid-glycolic acid copolymer, polyaspartic acid-polyethylene glycol-carboxyl, and amino or carboxyl compound modified nanomaterial.

A continuous analysis sensor, comprising a substrate, an electrode layer arranged on the substrate, an enzyme-sensing membrane arranged on the electrode layer, and a biocompatible outer membrane arranged on the enzyme-sensing membrane. The enzyme-sensing membrane is a NAD (P) immobilized enzyme-sensing membrane prepared by using the above immobilization method.

The present invention first provides a NAD(P) modification method. In this method, Br-(CH₂)ₓ-NH₂·HBr is used to react with NAD(P) (i.e. nicotinamide adenine dinucleotide or nicotinamide adenine dinucleotide phosphate) to obtain N¹-(x-(CH₂)ₓNH₂)-NAD(P)⁺, which then undergoes a rearrangement reaction to obtain N⁶-(x-(CH₂)ₓNH₂)-NAD(P)⁺. The N⁶-(x-(CH₂)ₓNH₂)-NAD(P)⁺ is prepared by modifying and adding an amino group on the NAD(P). Compared with the unmodified NAD(P), the prepared N⁶-(x-(CH₂)ₓNH₂)-NAD(P)⁺ is easily immobilized and connected to the carrier material, so that effective immobilization is achieved. Moreover, once immobilized, it is difficult for N⁶-(x-(CH₂)ₓNH₂)-NAD(P)⁺ to permeate through the polymer restricting membrane, thus ensuring that the concentration of NAD(P) in the sensor system remains at a high level during continuous monitoring. This effectively enhances the stability of the sensor and prolongs the functional life of the sensor. In addition, the modification method of the present invention uses Br-(CH₂)ₓ-NH₂·HBr (x=2-6) as the reagent, compared to introducing an amino group onto NAD(P) by using ethyleneimine for alkylation, the modification method of the present invention is more environmentally friendly, harmless and suitable for mass production.

The modification method of the present invention is illustrated in the following figures by taking x=2, namely, using Br-(CH₂)₂-NH₂·HBr (bromoethylamine hydrobromide) as an example.

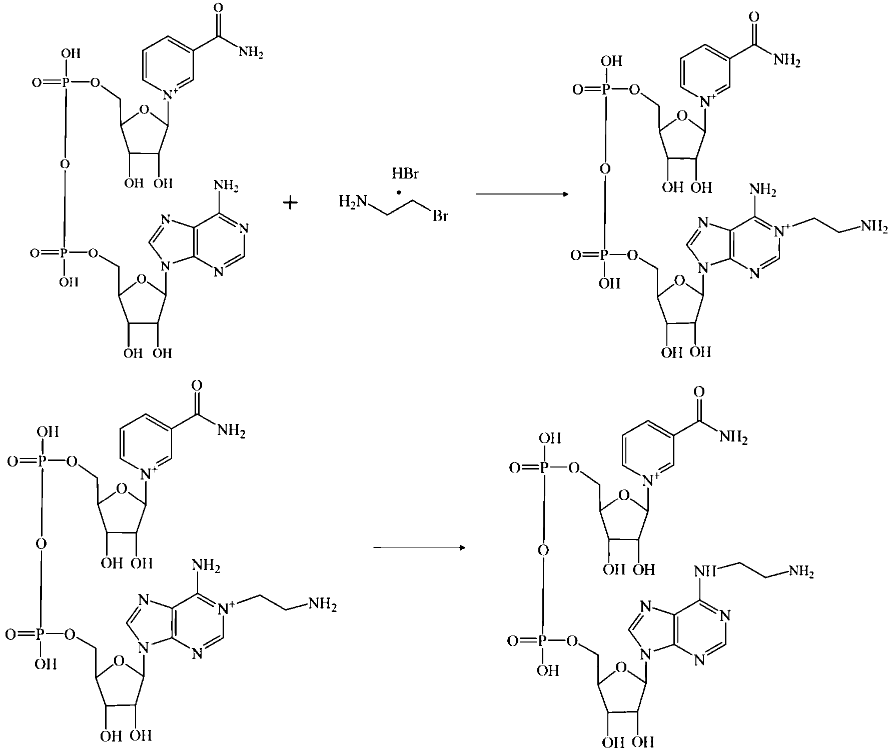

Preferably, according to the modification method of the present invention, in S1, respectively dissolving Br-(CH₂)ₓ-NH₂·HBr and NAD(P) in a first solvent, dropwise adding the Br-(CH₂)ₓ-NH₂·HBr solution into the NAD(P) solution, reacting at a temperature of 20~30°C for 18-24 hours under the protection of inert gas, and separating, thereby obtaining N¹-(x-(CH₂)ₓNH₂)-NAD(P)⁺; in S2, dissolving N¹-(x-(CH₂)ₓNH₂)-NAD(P)⁺ in water, adding into 1-5mM LiOH solution, adjusting the pH to 6.4-6.6, reacting at a temperature of 45-55°C for 4-6 hours, and separating, thereby obtaining N⁶-(x-(CH₂)ₓNH₂)-NAD(P)⁺; preferably, dissolving Br-(CH₂)ₓ-NH₂·HBr in methanol, and dissolving NAD(P) in water.

Br-(CH₂)ₓ-NH₂·HBr used in the present invention is preferably any one of bromoethylamine hydrobromide, bromopropylamine hydrobromide, bromobutylamine hydrobromide, bromopentylamine hydrobromide, and bromhexylamine hydrobromide. The prepared N⁶-(x-(CH₂)ₓNH₂)-NAD(P)⁺ is respectively N⁶-(2-(CH₂)₂NH₂)-NAD(P)⁺, N⁶-(3-(CH₂)₃NH₂)-NAD(P)⁺, N⁶-(4-(CH₂)₄NH₂)-NAD(P)⁺, N⁶-(5-(CH₂)₅NH₂)-NAD(P)⁺, and N⁶-(6-(CH₂)₆NH₂)-NAD(P)⁺. More preferably, N⁶-(2-(CH₂)₂NH₂)-NAD(P)⁺, N⁶-(3-(CH₂)₃NH₂)-NAD(P)⁺ and N⁶-(4-(CH₂)₄NH₂)-NAD(P)⁺ are respectively prepared by using bromoethylamine hydrobromide, bromopropylamine hydrobromide, and bromobutylamine hydrobromide.

The method of the present invention may also be used for the modification of NAD(P)-NH₂. Although NAD(P)-NH₂ itself has an amino group, its activity is low, making it still difficult to be immobilized. However, after using the method of the present invention for modification, the activity for connecting to the carrier material is significantly improved, especially for the products obtained by using bromoethylamine hydrobromide, bromopropylamine hydrobromide, or bromobutylamine hydrobromide to react with NAD(P)-NH₂.

In the present invention, preferably, the first solvent is water and/or methanol. More preferably, Br-(CH₂)ₓ-NH₂·HBr is dissolved in methanol, and NAD(P) is dissolved in water.

The present invention also provides a method for immobilizing modified NAD(P). The N⁶-(x-(CH₂)ₓNH₂)-NAD(P)⁺ is coupled and immobilized onto the carrier material with amino or carboxyl functional groups. Amide bonds are formed between the modified NAD(P) and the carrier material, and NAD(P) is firmly immobilized on the carrier material by covalent bonds, thereby effectively reducing the permeation.

Preferably, the carrier material is any one of polylysine, polyethyleneimine, polylactic acid-glycolic acid copolymer, polyaspartic acid-polyethylene glycol-carboxyl, and amino or carboxyl compound modified nanomaterial. The amino compound-modified nanomaterial may be polyethyleneimine-modified nano-silica, another nano-material having an amino group, or carboxyl compound-modified nano-silica, etc.

Nanomaterials possess an extremely large specific surface area, enabling them to adsorb enzymes and create a relatively milder reaction microenvironment around the enzyme proteins. This ensures that the enzymes always react under optimal conditions and are free from external environmental interference, thereby enhancing their stability. Additionally, nanomaterials can introduce nanoscale effects into biocatalysts, making enzyme proteins more reactive and thereby increasing their catalytic activity. Furthermore, nanomaterials modified with amino groups can covalently immobilize NAD(P) through amide bond formation. This not only improves the stability and catalytic activity of the enzymes but also prevents the coenzyme from permeating, facilitating the long-term stable operation of sensor systems.

Nanomaterials modified with amino or carboxyl compounds may be obtained by purchasing from the market or preparing using the methods in the prior art.

When coupling and immobilizing the modified N⁶-(x-(CH₂)ₓNH₂)-NAD(P)⁺ to the carrier material with amino or carboxyl functional groups, methods known in the field may be used. When it is coupled to the carrier material with amino functional groups, a coupling agent (such as glutaraldehyde, or PEGDGE-400) may be added to assist the process.

The NAD(P) modification method, immobilization method and the prepared immobilized enzyme-sensing membrane of the present invention are applicable to all sensors that require NAD(P) as a coenzyme for continuous and stable analysis, for example, sensors for detecting glucose (glucose-reaction enzyme), lactate (lactate-reaction enzyme), or β-hydroxybutyrate (ketone-reaction enzyme).

In the present invention, ketone reaction enzyme is taken as an example. The enzyme-sensing layer mainly comprises β-hydroxybutyrate dehydrogenase that responds to β-hydroxybutyrate, the coenzyme that is operatively in contact with the β-hydroxybutyrate dehydrogenase, diaphorase that enables the regeneration of the coenzyme, and the electron mediator. These components are combined to prepare the continuous analysis sensor. The coenzyme may adopt N⁶-(x-(CH₂)ₓNH₂)-NAD(P)⁺ of the present invention or N⁶-(x-(CH₂)ₓNH₂)-NAD(P)⁺ immobilized on the carrier material with amino functional groups.

### BRIEF DESCRIPTION OF DRAWINGS

To more clearly illustrate the technical solutions of the embodiments in this invention or the prior art, the drawings used in the description of the embodiments or the prior art are briefly introduced below. Obviously, the drawings described below are merely some of the embodiments of this invention. For those skilled in the art, other drawings may be obtained without paying creative labor.
Figure 1 is a schematic diagram illustrating the response current - test concentration curves of each sensor system prepared in embodiment 4 of the present invention;
Figure 2 is a schematic diagram illustrating the response current - test duration curves of each sensor system prepared in embodiment 4 of the present invention; and
Figure 3 is a schematic diagram illustrating the response current - test concentration curves of the stability test of each sensor system prepared in embodiment 4 of the present invention.

### DETAILED DESCRIPTION OF THE INVENTION

While specific embodiments of the invention have been described in detail, it will be appreciated by those skilled in the art that various modifications and alternatives to those details could be developed in light of the overall teachings of the disclosure. Accordingly, the particular arrangements disclosed are meant to be illustrative only and not limiting as to the scope of the invention which is to be given the full breadth of the claims appended and any and all equivalents thereof.

The difference between NAD and NADP lies solely in that NADP has one more phosphate group than NAD, as shown in the chemical structures below. In step 1 (S1) of the modification process for NAD or NADP, the reaction involves the alkylation of the adenine ring using an alkylating agent Br-(CH₂)x-NH₂·HBr, and in step 2 (S2), the reaction is a Dimroth rearrangement reaction, which does not involve the phosphate group on NADP. In conclusion, the phosphate group on NADP does not participate in the reactions of S1 and S2, nor does it participate in the subsequent coupling reaction. The phosphate group on NADP does not affect the modification and immobilization effects in this invention. Therefore, both NAD and NADP are suitable for the modification and immobilization methods described herein, and the prepared enzyme-sensing membranes exhibit identical technical effect.

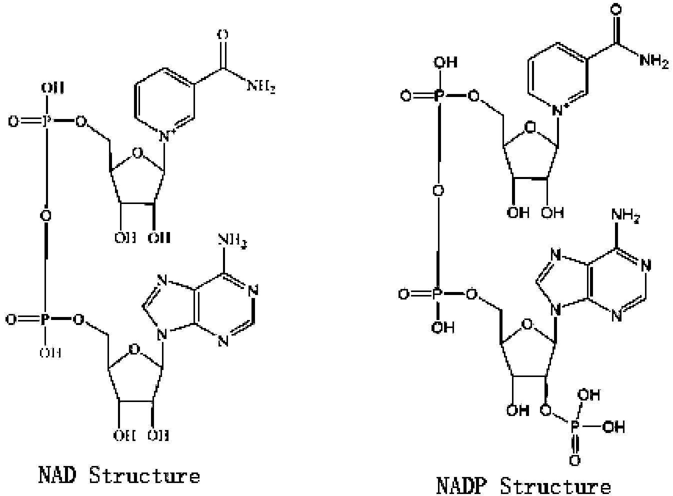

Based on the above, in this invention, the experimental effect is verified by using NAD. The experimental effect of NADP is equivalent to that of NAD. The details are in the following embodiments.

### Embodiment 1: synthesis of N⁶-(2-(CH₂)₂NH₂)-NAD⁺

S1: dissolving 1.2g (gram) of bromoethylamine hydrobromide in 2mL (mini liter) of methanol, dissolving 4.8g of NAD in 5mL of water, dropwise adding the bromoethylamine hydrobromide solution into a flask containing the NAD solution, and reacting at a temperature of 25°C for 20 hours under nitrogen protection; after the reaction is completed, performing vacuum rotary evaporation to remove the solvent, and washing with 5mL of ethanol for three times; performing centrifugal precipitation, and performing vacuum rotary evaporation for drying, thereby obtaining a white powder; performing rotary evaporation concentration on the collected components after the ion exchange chromatography, thereby obtaining N¹-(2-(CH₂)₂NH₂)-NAD⁺, wherein the yield is 47.8% (2.3g);
S2: taking 2.0g of N¹-(2-(CH₂)₂NH₂)-NAD⁺ obtained in S1 and dissolving in 5mL of deionized water; adding into a 1mM LiOH solution, adjusting the pH to 6.5, and reacting at a temperature of 50°C for 5 hours; after the reaction is completed, performing vacuum rotary evaporation for drying, thereby obtaining a white powder; performing rotary evaporation concentration on the collected components after the ion exchange chromatography, thereby obtaining N⁶-(2-(CH₂)₂NH₂)-NAD⁺, wherein the yield is 62% (1.24g).

### Embodiment 2: immobilization of N⁶-(2-(CH₂)₂NH₂)-NAD⁺

The N⁶-(2-(CH₂)₂NH₂)-NAD⁺ prepared in embodiment 1 is immobilized onto a polylysine polymer conjugated with an electron mediator by using a coupling agent glutaraldehyde.

### Embodiment 3: immobilization of N⁶-(2-(CH₂)₂NH₂)-NAD⁺

The N⁶-(2-(CH₂)₂NH₂)-NAD⁺ prepared in embodiment 1 is immobilized onto a silica nanocarrier material having a size of 300-500nm modified with polyethyleneimine by using the coupling agent glutaraldehyde.

The products prepared in embodiment 1 and embodiment 3, as well as unmodified NAD (as control 1), are used to prepare a blood ketone sensor system according to the materials listed in Table 1.

**Table 1**

| Embodiment 1 | Embodiment 3 | Control 1 |
|---|---|---|
| N⁶-(2-(CH₂)₂NH₂)-NAD⁺ | The SiO₂ nanocarrier material for immobilizing N⁶-(2-(CH₂)₂NH₂)-NAD⁺ | NAD |
| β-hydroxybutyrate dehydrogenase | β-hydroxybutyrate dehydrogenase | β-hydroxybutyrate dehydrogenase |
| Diaphorase | Diaphorase | Diaphorase |
| Electron mediator | Electron mediator | Electron mediator |
| Cross-linking agent | Cross-linking agent | Cross-linking agent |
| HEPES buffer solution | HEPES buffer solution | HEPES buffer solution |

A β-hydroxybutyrate-sensitive layer solution is prepared according to the materials listed in Table 1. The β-hydroxybutyrate-sensitive layer solution is deposited onto an electrode layer to obtain an enzyme-sensing membrane. Subsequently, a permeation-restricting membrane solution is coated on the enzyme-sensing membrane to form a biocompatible outer membrane, thereby obtaining the sensor system.

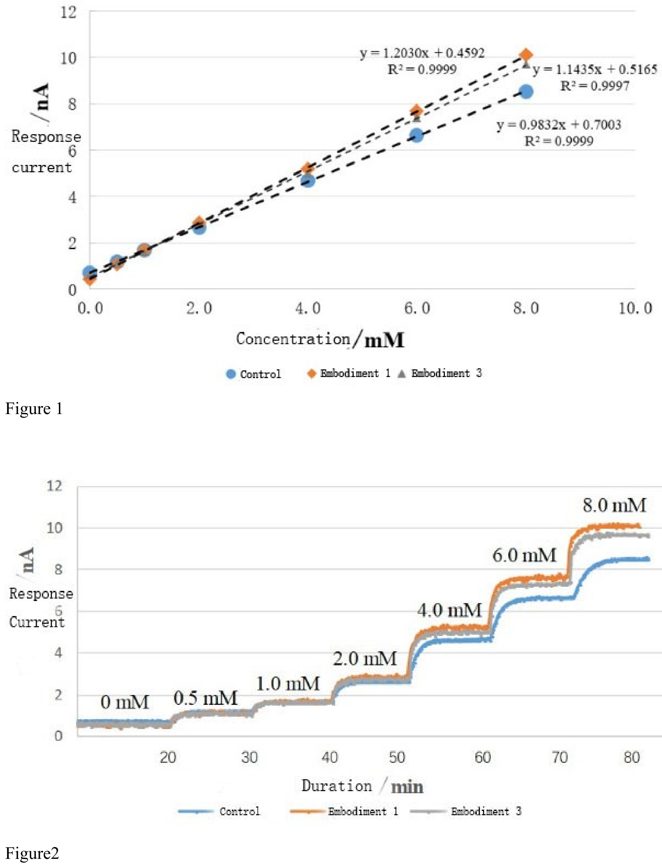

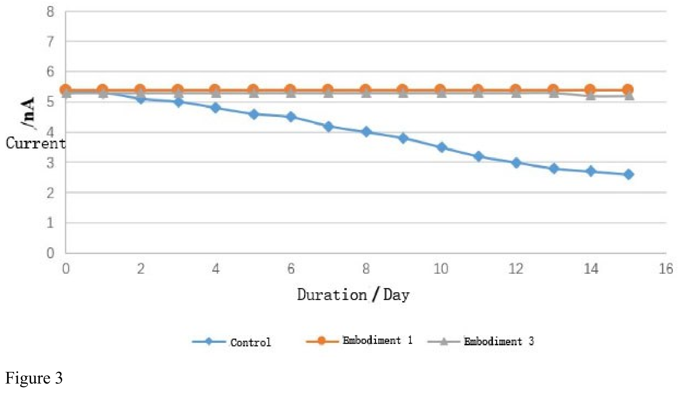

After performing separate tests on the above sensor systems, the linear response current - test concentration curve is shown in Figure 1, the response current - test duration curve is shown in Figure 2, and the response current - test concentration curve of the stability test is shown in Figure 3.

As shown in Figure 1, the responses of each sensor system at a temperature of 37°C under β-hydroxybutyrate concentrations of 0mM, 0.5mM, 1.0mM, 2.0mM, 4.0mM, 6.0mM and 8.0mM are measured. The R² values of the response current linear curves for β-hydroxybutyrate are all greater than 0.98, indicating that both the immobilized NAD and the non-immobilized NAD sensors have good linear relationships. Therefore, the immobilized NAD does not compromise the linear relationship between current and concentration.

However, as can be seen from Figure 2, the response current of the control 1 (unmodified NAD) decreases significantly after 50 minutes of testing, indicating that the unmodified NAD permeates through the sensor system over time. This compromises the catalytic reaction capability of the sensor system and affects the detection results.

Similarly, as shown in Figure 3, stability tests are performed. The control 1 (unmodified NAD) cannot maintain 15-day stability at a temperature of 37°C in 8mM β-hydroxybutyrate, and the performance degradation is observed as early as the second day. In contrast, the products prepared in embodiments 1 and 3, which respectively use unmodified NAD, modified NAD and immobilized modified NAD sensors, are capable of maintaining stability for 15 days. This achieves longer-term stability of the blood ketone sensor, thereby prolonging the functional life of the blood ketone sensor.

The above description of the embodiments allows those skilled in the art to implement or use the present invention. Various modifications of these embodiments will be apparent to those skilled in the art. The general principles defined herein may be implemented in other embodiments without departing from the spirit or scope of the present invention. Therefore, the present invention will not be limited to the described embodiments, but will be within the broadest scope consistent with the principles and novel features disclosed herein.

## Claims

1. A NAD(P) modification method, comprising:
S1: using Br-(CH₂)ₓ-NH₂·HBr to react with NAD(P), thereby obtaining N¹-(x-(CH₂)ₓNH₂)-NAD(P)⁺;
S2: performing rearrangement reaction on N¹-(x-(CH₂)ₓNH₂)-NAD(P)⁺, thereby obtaining
N⁶-(x-(CH₂)ₓNH₂)-NAD(P)⁺, wherein x=2-6.

2. The modification method of claim 1, wherein S1 comprising: respectively dissolving Br-(CH₂)ₓ-NH₂·HBr and NAD(P) in a first solvent; subsequently, dropwise adding the Br-(CH₂)ₓ-NH₂·HBr solution into the NAD(P) solution; reacting at a temperature of 20-30°C for 18-24 hours under the protection of inert gas , and separating, thereby obtaining N¹-(x-(CH₂)ₓNH₂)-NAD(P)⁺.

3. The modification method of claim 2, wherein the first solvent is water and/or methanol, and wherein the inert gas is nitrogen or helium.

4. The modification method of claim 2, wherein S1comprising: after the reaction is completed, performing vacuum rotary evaporation to remove solvent, washing with ethanol for 2-3 times, centrifuging for precipitation, and performing vacuum rotary for drying; after performing ion exchange chromatography, obtaining N¹-(x-(CH₂)ₓNH₂)-NAD(P)⁺.

5. The modification method of claims 1-4, wherein S2 comprising: dissolving N¹-(x-(CH₂)ₓNH₂)-NAD(P)⁺ in water, adding into 1-5mM LiOH solution, adjusting the pH to 6.4-6.6, reacting at a temperature of 45-55°C for 4-6 hours, and separating, thereby obtaining N⁶-(x-(CH₂)ₓNH₂)-NAD(P)⁺.

6. The modification method of claim 5, wherein S2 comprising: after the reaction is completed, performing ion exchange chromatography, and then performing vacuum rotary evaporation for drying, thereby obtaining N⁶-(x-(CH₂)ₓNH₂)-NAD(P)⁺.

7. The modification method of claim 1, wherein Br-(CH₂)ₓ-NH₂·HBr is specifically any one of bromoethylamine hydrobromide, bromopropylamine hydrobromide, bromobutylamine hydrobromide, bromopentylamine hydrobromide, and bromhexylamine hydrobromide.

8. A modified NAD(P) immobilization method, comprising: coupling and immobilizing N⁶-(x-(CH₂)ₓNH₂)-NAD(P)⁺ of claims 1-7 to a carrier material having amino or carboxyl functional groups, wherein x=2-6.

9. The immobilization method of claim 8, wherein the carrier material is any one of polylysine, polyethyleneimine, polylactic acid-glycolic acid copolymer, polyaspartic acid-polyethylene glycol-carboxyl, and amino or carboxyl compound modified nanomaterial.

10. A continuous analysis sensor, comprising:
a substrate,
an electrode layer arranged on the substrate,
an enzyme-sensing membrane arranged on the electrode layer, and
a biocompatible outer membrane arranged on the enzyme-sensing membrane, wherein the enzyme-sensing membrane is a NAD(P) immobilized enzyme-sensing membrane prepared by using the immobilization method of claims 8-9.
